# EUROPEAN PATENT APPLICATION

(11) **EP 1 034 799 A1**
(43) Date of publication of application: **13.09.2000**
(21) Application number: 99103907.4
(22) Date of filing: 05.03.1999
(51) Int. Cl.: A61L 15/46

(54) **Articles having an odour control system comprising a water soluble oxidising agent and an emulsifier**

(71) Applicant: THE PROCTER & GAMBLE COMPANY, Cincinnati, Ohio 45202 (US)
(72) Inventor: Carlucci, Giovanni, 66100 Chieti (IT); Di Cintio, Achille, 65126 Pescara (IT); Pesce, Antonella, 65120 Pescara (IT)
(74) Representative: Kremer, Véronique Marie Joséphine

(57) **Abstract**

The present invention relates to articles, preferably absorbent articles like sanitary napkins and pantiliners, for controlling odour typically associated with bodily fluids, which comprise a water soluble oxidising agent together with an emulsifier, preferably a surfactant having an (hydrophilic/lipophilic balance) HLB of less than 12. In a preferred embodiement the articles further comprise an additional odour control agent.

## Description

### Field of the Invention

This invention relates to articles, such as absorbent articles, for controlling odour, especially those associated with bodily fluids, comprising a water soluble oxidising agent together with an emulsifier.

### Background of the Invention

Malodours may be present in the environment from numerous sources both animate and inanimate. Many products and articles are available which aim to avoid or minimise the detection of such odours. In particular, it is particularly desirable to provide odour control materials to address the malodours which are generated by the human body, or from bodily fluids such as perspiration, urine, faeces, menstrual fluids, vaginal fluids and the like.

Articles like absorbent articles for example are designed to be worn by humans to absorb bodily fluids, such as urine, menstrual fluid and perspiration, etc. Examples of absorbent articles include sanitary napkins, pantiliners, disposable diapers, incontinence pads, tampons, perspiration pads, and the like.

In use, the absorbent articles are known to acquire a variety of compounds, for example volatile fatty acids (e.g. isovaleric acid), ammonia, amines (e.g. triethylamine), sulphur containing compounds (e.g. mercaptans, sulphides), alcohols, ketones and aldehydes (e.g., furaldehyde) which release unpleasant odours. These compounds may be present in the bodily fluid or may be developed by chemical reactions and/or any fluid degradation mechanisms once the bodily fluid is absorbed into the absorbent article like for example a feminine pad. In addition bodily fluids usually contain microorganisms and/or enzymes that can also generate malodorous by products as a result of degradation mechanisms like putrefactive degradation, acid degradation, proteins degradation, fat degradation and the like. Unpleasant odours which emanate from absorbent pads when in use may make the wearer feel self conscious.

Various odour control materials have been disclosed in the art to combat some of the unpleasant odours referred to above. Indeed solutions have been provided that use different technical approaches like masking, i.e., covering the odour with a perfume, or absorbing the odour already present in the bodily fluids and those generated after degradation.

Most of the focus in the prior art is found on the odour absorption technology. Examples of these types of compounds include activated carbons, clays, zeolites, silicates, starches, cyclodextrine, ion exchange resins and various mixture thereof as for example described in EP-A-348 978, EP-A-510 619, WO 91/12029, WO 91/11977, WO89/02698, and/or WO 91/12030. All of these types of odour absorbing agents are believed to control odour by mechanisms whereby the malodorous compounds and their precursors are physically absorbed by the agents and thus such agents hinder the exit of the odour from articles like absorbent articles. However, such mechanisms are not completely effective as the formation of the odour itself is not prevented and thus odour detection is not completely avoided.

Thus although these materials provide some control of odours associated with bodily fluids, there still exists a need of further improvement in terms of odour control over a wider range of malodorous compounds.

It is an object of the present invention to provide effective odour control over a wider range of malodorous compounds. More particularly, it is an object of the present invention to provide articles, especially disposable absorbent articles, which deliver a broader spectrum of odour control.

It has now been found that the above needs can be addressed by combining a water soluble oxidising agent together with an emulsifier, preferably a surfactant having a hydrophilic/lipophilic balance (HLB) of less than 12, as the odour control system for an article coming into contact with odours, especially those associated with bodily fluid.

It has been surprisingly discovered that the combination of a water soluble oxidising agent, preferably a persulfate or urea peroxide, together with an emulsifier, preferably a surfactant having an HLB of less than 12, in an article, like an absorbent article coming into contact with bodily fluids, results in significantly improved odour control (i.e., reduced malodor detection or even malodour prevention), as compared to the odour control obtained with the same article comprising only the oxidising agent as the odour control system. Indeed, this combination gives effective odour control over a broader malodorous range from water soluble malodorous components to non water soluble malodorous components.

It is speculated that the odour control system of the present invention prevents the formation of odour by for example blocking the enzymatic and/or microbial activity as well as combats the odorous components already present by oxidising them into no-smelling components.

Indeed, it is speculated that the water soluble oxidising agents herein oxidise sensitive sulphidryl and sulphur bonds typically present in enzymes, thereby deactivating the enzymes which otherwise would have contributed to the normal metabolism of the micro-organisms. It is further speculated that these water soluble oxidising agents oxidise most malodorous water soluble components into less volatile ones (e.g., sulphidic acid compounds into sulphoxides, chetones into esters)

It is further believed that the emulsifier herein acts as an antimicrobial compound too. Indeed it has been observed that the emulsifiers herein, especially the surfactants with an HLB below 12 are able to disrupt the chemiosmotic function of the lipoprotein cytoplasmatic membrane of the microbe/bacteria cells and thus disrupt the transport function at the cell walls.

Furthermore it is speculated that the emulsifier herein also acts as a carrier for the water soluble oxidising agent as described herein and thus contributes to bring the oxidising agent into closer contact with the non water soluble oxidable malodorous components present in the bodily fluid. Actually, the presence of the emulsifier results in significantly improved effectiveness of the oxidising odour control agent, especially versus non water soluble oxidable malodorous components. In other words, the present combination allows odour control of malodorous components ( e.g., fatty acids with a carbon chain of more than 7 carbon atoms) which would otherwise not have been controlled by the water soluble oxidising agent in absence of the emulsifier.

A further advantage associated with the absorbent articles of the present invention is that they deliver a better feeling and more acceptable cleanness level for the person wearing them. Indeed, the present invention due to the presence of the water soluble oxidizing agent as described herein provides an absorbent article capable of changing the color of the menstruation (red blood color) to a pale red color and even to a whitish color.

Whereas the present invention is preferably directed to absorbent articles like pantiliners, feminine napkins, incontinent pads, diapers, tampons, interlabial pads, perspiration pads, surgical pads, breast pads and the like, other articles may include the odour control system as described herein too for the purpose of effective odour control especially when in contact with bodily fluids. Such other applications include other articles designed to be worn in contact with the body such as clothing, bandages, thermal pads, acne pads, cold pads, compresses, surgical pads/dressings and the like, body and household cleansing articles like impregnated wipes (e.g. baby wipes, wipes for feminine intimate hygiene), impregnated tissues, towels, articles for absorbing perspiration such as shoe insoles, shirt inserts, carpets and the like, and articles for animals like litters and the like.

### Background art of the invention

US 4363322 discloses deodorising and disinfecting liquid-absorbing products such as a sanitary napkin, a compress or a diaper, comprising a liquid absorbing material and inside the product at a distance from its outer edges a substance which gives off oxygen in contact with moisture like peroxides, ozonides, superoxides, oxo-ozonides and the like.

EP-A-268 459 discloses a body fluid absorbing article provided with one absorbent member comprising 50% to 99% by weight of a fibrous material and 50% to 1% by weight of an absorbent polymer, which absorbent member contains at least one compound selected from sulphur-containing reducing agents, antioxidants and oxidising agents.

None of these references disclose the presence of the emulsifier according to the present invention, let alone its combination with a water soluble oxidising agent for outstanding odour control in an article, like a disposable absorbent article, when it comes into contact with bodily fluids.

### Summary of the Invention

The present invention relates to an article, preferably a disposable absorbent article, for controlling odour typically associated with bodily fluids, comprising at least one water soluble oxidising agent and at least one emulsifier. In a preferred embodiment of the invention the article also comprises an additional odour control agent, preferably at least an absorbing gelling material.

### Detailed description of the Invention

The articles according to the present invention, for controlling odours, especially those associated with bodily fluids, comprise as an essential element a water soluble oxidising agent and an emulsifier.

By "article" it is meant herein any tridimentional solid material being able to receive/carry an odour control system as described herein after. Preferred articles according to the present invention are disposable absorbent articles that are designed to be worn in contact with the body of a user and to receive fluids discharged from the body, such as disposable absorbent pantiliners, sanitary napkins, catamenials, incontinence inserts/pads, diapers, tampons, interlabial pads/inserts, breast pads and the like. Other articles suitable according to the present invention also include other articles designed to be worn in contact with the body such as clothing, bandages, thermal pads, acne pads, cold pads, compresses, surgical pads/dressings and the like, articles for absorbing perspiration such as shoe insoles, shirt inserts, perspiration pads and the like, body and household cleansing articles like impregnated wipes (e.g. baby wipes, wipes for feminine intimate hygiene), impregnated tissues, towels, and the like, and articles for animals like litters and the like.

By "bodily fluids" it is meant herein any fluid produced by human or animal body occurring naturally or accidentally like for instance in the case of skin cutting, including for instance perspiration, urine, menstrual fluids, faeces, vaginal secretions and the like.

### The water soluble oxidising agent

The water soluble oxidising agents according to the present invention are any oxidising agent known to those skilled in the art that have a solubility in water of more than 0.3 g per 100 ml of water at a temperature of 25°C.

Typically the water soluble oxidising agents according to the present invention have a solubility in water of more than 0.4 g per 100 ml of water at 25°C and more preferably of more than 0.5 g per 100 ml of water.

The water soluble oxidising agents for use herein include oxygen bleaches and/or hypohalite bleaches meeting to the solubility criteria defined in the present invention.

Hypohalite bleaches may be provided by a variety of sources, including bleaches that are oxidative bleaches and subsequently lead to the formation of positive halide ions as well as bleaches that are organic based sources of halides such as chloroisocyanurates.

Suitable hypohalite bleaches for use herein include the alkali metal and alkaline earth metal hypochlorites, hypobromites, hypoiodites, dichloroisocyanurates (e.g., potassium and sodium dichloroisocyanurates), trichlorocyanurates (e.g., potassium and sodium trichlorocyanurates), N-chloroimides, N-chloroamides, N-chloroamines, chlorohydantoins, chlorinated trisodium phosphate dodecahydrates, chloramine T, chlorine dioxide, chlorine, chlorite, perchlorates, alkali metal salts thereof or alkaline earth metal salts thereof or mixture thereof.

The preferred hypohalite bleaches amongst the above described are the alkali metal and/or alkaline earth metal hypochlorites such as lithium hypochlorite, calcium hypochlorite, potassium hypochlorite, magnesium hypochlorite, as well as other hypohalite bleaches like chlorinated trisodium phosphate dodecahydrate, potassium dichloroisocyanurate, sodium dichloroisocyanurate, potassium trichlorocyanurate, sodium trichlorocyanurate, other alkali metal salts or mixtures thereof.

Suitable oxygen bleaches for use herein include peroxygen bleaches or mixtures thereof.

Such peroxygen bleaches include percarbonates, persilicates, persulphates, perphosphates, perborates, alkyl hydroperoxides, peroxides, urea peroxides, periodates, salts thereof or mixtures thereof.

Suitable alkyl hydroperoxides for use herein include tert-butyl hydroperoxide, cumyl hydroperoxide, 2,4,4-trimethylpentyl-2-hydroperoxide, di-isopropylbenzene-monohydroperoxide, tert-amyl hydroperoxide and 2,5-dimethyl-hexane-2,5-dihydroperoxide.

Suitable peroxides for use herein include for example lithium peroxide, sodium peroxide, other alkali metal salts thereof or alkaline earth metal salts thereof or mixture thereof.

Suitable perphosphates for use herein include for example lithium perphosphate, sodium perphosphate, potassium perphosphate, zinc perphosphate, ammonium perphosphate, calcium perphosphate, barium perphosphate, magnesium perphosphate, other alkali metal salts thereof or alkaline earth metal salts thereof or mixture thereof.

Suitable perborates for use herein include for example sodium perborate, potassium perborate, ammonium perborate or other alkali metal salts thereof or alkaline earth metal salts thereof or mixture thereof

Suitable percarbonates for use herein include for example sodium percarbonate, potassium percarbonate, ammonium percarbonate or other alkali metal salts thereof or alkaline earth metal salts thereof or mixture thereof.

Suitable persulphates for use herein include sodium persulphate, potassium dipersulphate, potassium persulphate, ammonium persulphate as well as other alkali metal salts thereof or mixture thereof. Suitable persulfate salts or mixtures thereof to be used herein according to the present invention, also includes monopersulfate triple salt. One example of monopersulfate salt commercially available is potassium monopersulfate commercialized by Peroxide Chemie GMBH under the trade name Curox®. Other persulfate salts such as dipersulfate salts commercially available from Peroxide Chemie GMBH can be used in the emulsions according to the present invention.

Other suitable peroxygen bleaches also include O,O,-t-buthyl-O-isopropyl mono-peroxycarbonate, 2-ethylhexyl percarbonate and sec-butyl percarbonate and corresponding perborates as well as corresponding persulphates.

Further suitable peroxygen bleaches also include inorganic oxides like, potassium permanganate, ruthenium tetra-oxide, osmium tetra-oxide, chromium compounds like chromate salts, dichromate salts (e.g., sodium and potassium dichromate), lead compounds like lead tetracetate, nitrate compounds, metallic salts like ferric and cupric salts (e.g., cupric sulphate, cupric chloride, ferric chloride) as well as mixtures thereof.

Persulphates and urea peroxides are preferred herein as the water soluble oxidizing agents. Urea peroxide may be commercially available from the FLUKA under the name Hydrogen Peroxide Urea Adduct or Carbamide Perhydrate.

These water soluble oxidising agents have a dual mechanism: they prevent the formation of odour by for example blocking enzymatic and/or microbial activity and they combat the malodorous compounds already present by oxidising them into non-smelling compounds.

Typically, the articles according to the present invention like disposable absorbent articles comprise the water soluble oxidizing agent or a mixture thereof at a level of from 1 gm⁻² to 500 gm⁻², preferably from 5 to 250 gm⁻², more preferably from 10 gm⁻² to 100 gm⁻² and most preferably from 20 gm⁻² to 70gm⁻²

### The emulsifier

The articles of the present invention comprise as an essential element of the odour control system an emulsifier or a mixture thereof.

By 'emulsifier' it is meant herein any compound able to enhance the lyposolubility of the water soluble oxidising agent as described herein.

Suitable emulsifiers for use herein include surfactants having a hydrophilic/lipophilic balance (HLB) of less than 12, or a mixture thereof.

As used herein, the term 'surfactant' refers to any compound or mixture of compounds that reduces surface tension when dissolved or dispersed in water or water solutions, or which reduces interfacial tension between two liquids or between a liquid and a solid.

As used herein, the term 'hydrophilic/lipophilic balance (HLB)' refers to the HLB numbering system developed at the Atlas Powder Company, and now used worldwide, to rank surfactant by relative water or oil solubility. In general, compounds represented by numbers below about 12 are oil soluble, those above about 12 are water soluble. A more complete description of the HLB system is found on page 479 et seq. Of Surface Active Agents and detergents, Volume II, (interscience Publishers, Inc., 1958).

Preferred surfactants for use herein are those having an HLB of less than 10 and the more preferred ones are those having an HLB of less than 9 or even less than 8.

Suitable surfactants having an HLB of less than 12 for use herein are generally nonionic ones, anionic ones and/or zwitterionic ones. A listing of surfactants having an HLB of less than 12 can be found in McCutcheon's Emulsifiers and Detergents, International or North American edition. Highly preferred surfactants for use herein are the nonionic surfactants.

Typically, the articles according to the present invention, like disposable absorbent articles, comprise the emulsifier or a mixture thereof at a level of from 0.5 gm⁻² to 200 gm⁻², preferably from 5 gm⁻² to 100 gm⁻², more preferably from 10 gm⁻² to 60 gm⁻².

### Nonionic surfactants

Suitable nonionic surfactants for use herein are those with an HLB (hydrophilic lipophilic balance) of less than 12, preferably those with an HLB of less than 10 and most preferably those with an HLB of less than 9.

Suitable nonionic surfactants for use herein include alkoxylated fatty alcohols preferably, fatty alcohol ethoxylates and/or propoxylates. Indeed, a great variety of such alkoxylated fatty alcohols are commercially available which have very different HLB values (hydrophilic lipophilic balance). The HLB values of such alkoxylated nonionic surfactants depend essentially on the chain length of the fatty alcohol, the nature of the alkoxylation and the degree of alkoxylation. The nonionic surfactants suitable to be used herein tend to have a low degree of alkoxylation and a long chain fatty alcohol. Surfactants catalogs are available which list a number of surfactants including nonionics, together with their respective HLB values.

Suitable chemical processes for preparing the nonionic surfactants for use herein include condensation of corresponding alcohols with alkylene oxide, in the desired proportions. Such processes are well known to the man skilled in the art and have been extensively described in the art. As an alternative, a great variety of alkoxylated alcohols suitable for use herein is commercially available from various suppliers.

Preferred nonionic surfactants having an HLB of less than 12 to be used according to the present invention are the alkoxylated nonionic surfactants according to the formula RO-(C₂H₄O)ₙ₍C₃H₆O)ₘH, wherein R is a C₆ to C₂₄ alkyl chain or a C₆ to C₂₈ alkyl benzene chain, and wherein n+m is from 0.5 to 10 and n is from 0 to 10 and m is from 0 to 10, and preferably n+m is from 0.5 to 8 and, n and m are from 0 to 8. The preferred R chains for use herein are the C₈ to C₂₂ alkyl chains, straight or branched. Preferred nonionic surfactants for use in the compositions according to the invention are the condensation products of ethylene oxide with alcohols having a straight alkyl chain, having from 6 to 22 carbon atoms, wherein the degree of ethoxylation is from 1 to 10, preferably from 2 to 8.

Example of suitable nonionic surfactants for use herein are Dobanol® 91-2.5 (HLB= 8.1; R is a mixture of C₉ and C₁₁ alkyl chains, n is 2.5 and m is 0), or Lutensol® TO3 (HLB=8; R is a mixture of C₁₃ and C₁₅ alkyl chains, n is 3 and m is 0), or Tergitol® 25L3 (HLB= 7.7; R is in the range of C₁₂ to C₁₅ alkyl chain length, n is 3 and m is 0), or Dobanol® 23-3 (HLB=8.1; R is a mixture of C₁₂ and C₁₃ alkyl chains, n is 3 and m is 0), or Dobanol® 23-2 (HLB= 6.2; R is a mixture of C₁₂ and C₁₃ alkyl chains, n is 2 and m is 0), Tergitol ® 15-S-5 (HLB =10.6, R is a C11-C15 secondary alcohol, n is 5), Neodol® 23-6.5 (HLB=10, R is a C12-C13 alkyl chain and n is 6.5) or mixtures thereof. Preferred herein are Neodol® 13-6.5, Tergitol ® 15-S-5, Dobanol® 23-3, or Dobanol® 23-2, Lutensol® TO3, or mixtures thereof. The Dobanol® and Neodol® surfactants are commercially available from SHELL. The Lutensol® surfactants are commercially available from BASF and the Tergitol® surfactants are commercially available from UNION CARBIDE.

Other examples of suitable alkoxylated nonionic surfactants also include ethoxylated lauryl alcohol, ethoxylated cetyl alcohol, ethoxylated oleyl alcohol and/or ethoxylated stearyl alcohol commercially available from ICI under the name Brij®.

Other suitable nonionic surfactants to be used herein are non alkoxylated surfactants. An example is Dobanol® 23 (HLB<3 ).

Other suitable nonionic synthetic detergents include:
(i) the polyethylene oxide condensates of alkyl phenols, e.g., the condensation products of alkyl phenols having an alkyl group containing from about 6 to 12 carbon atoms in either a straight chain or branched chain configuration, with ethylene oxide, the said ethylene oxide being present in amounts equal to 5 to 15 moles of ethylene oxide per mole of alkyl phenol. The alkyl substituent in such compounds may be derived from polymerized propylene, diisobutylene, octane, and nonane;
(ii) those derived from the condensation of ethylene oxide with the product resulting from the reaction of propylene oxide and ethylene diamine products which may be varied in composition depending upon the balance between the hydrophobic and hydrophillc elements which is desired (HLB of less than 12). Examples are compounds containing from about 20% to about 60% polyoxyethylene by weight and having a molecular weight of from about 2000 to about 7000 resulting from the reaction of ethylene oxide groups with a hydrophobic base constituted of the reaction product of ethylene diamine and excess propylene oxide, said base having a molecular weight of the order of 1200 to 2000;
(iii) The condensation product of aliphatic alcohols having from 8 to 18 carbon atoms, in either straight chain or branched chain configuration, with ethylene oxide, e.g., a coconut alcohol ethylene oxide condensate having from 5 to 15 moles of ethylene oxide per mole of coconut alcohol, the coconut alcohol fraction having from 10 to 14 carbon atoms;
(iv)Trialkyl amine oxides and trialkyl phosphine oxides wherein one alkyl group ranges from 10 to 18 carbon atoms and two alkyl groups range from 1 to 3 carbon atoms; the alkyl groups can contain hydroxy substituents; specific examples are dodecyl di(2-hydroxyethyl)amine oxide and tetradecyl dimethyl phosphine oxide.

Also useful as a nonionic surfactant are the alkylpolysaccharides disclosed in U.S. Patent 4,565,647, Llenado, issued January 21, 1986, having a hydrophobic group containing from about 6 to about 30 carbon atoms, preferably from about 10 to about 16 carbon atoms and polysaccharide, e.g., a polyglycoside, hydrophilic group containing from about 1.3 to about 10, preferably from about 1.3 to about 3, most preferably from about 1.3 to about 2.7 saccharide units. Any reducing saccharide containing 5 or 6 carbon atoms can be used, e.g., glucose, galactose, and galactosyl moieties can be substituted for the glucosyl moieties. (Optionally the hydrophobic group is attached at the 2-, 3-, 4-, etc. positions thus giving a glucose or galactose as opposed to a glucoside or galactoside.) The intersaccharide bonds can be, e.g., between the one position of the additional saccharide units and the 2-, 3-, 4-, and/or 6- positions of the preceding saccharide units.

Optionally, and less desirably, there can be a polyalkyleneoxide chain joining the hydrophobic moiety and the polysaccharide moiety. The preferred alkyleneoxide is ethylene oxide. Typical hydrophobic groups include alkyl groups, either saturated or unsaturated, branched or unbranched containing from about 8 to about 18, preferably from about 10 to about 16, carbon atoms. Preferably, the alkyl group can contain up to about 3 hydroxy groups and/or the polyalkyleneoxide chain can contain up to about 10, preferably less than 5, alkyleneoxide moieties. Suitable alkyl polysaccharides are octyl, nonyldecyl, undecyldodecyl, tridecyl, tetradecyl, pentadecyl, hexadecyl, heptadecyl, and octadecyl, di-, tri-, tetra-, penta-, and hexaglucosides, galactosides, lactosides, glucoses, fructosides, fructoses and/or galactoses. Suitable mixtures include coconut alkyl, di-, tri-, tetra-, and pentaglucosides and tallow alkyl tetra-, penta-, and hexaglucosides.

The preferred alkylpolyglycosides have the formula:

R²O(CₙH₂ₙO)ₜ(glucosyl)ₓ

wherein R² is selected from the group consisting of alkyl, alkylphenyl, hydroxyalkyl, hydroxyalkylphenyl, and mixtures thereof in which the alkyl groups contain from about 10 to about 18, preferably from about 12 to about 14, carbon atoms; n is 2 or 3, preferably 2; t is from 0 to about 10, preferably 0; and x is from about 1.3 to about 10, preferably from about 1.3 to about 3, most preferably from about 1.3 to about 2.7. The glycosyl is preferably derived from glucose. To prepare these compounds, the alcohol or alkylpolyethoxy alcohol is formed first and then reacted with glucose, or a source of glucose, to form the glucoside (attachment at the 1-position). The additional glycosyl units can then be attached between their 1-position and the preceding glycosyl units 2-, 3-, 4- and/or 6-position, preferably predominantely the 2- position.

Although not preferred, the condensation products of ethylene oxide with a hydrophobic base formed by the condensation of propylene oxide with propylene glycol are also suitable for use herein. The hydrophobic portion of these compounds will preferably have a molecular weight of from about 1500 to about 1800 and will exhibit water insolubility. The addition of polyoxyethylene moieties to this hydrophobic portion tends to increase the water solubility of the molecule as a whole, and the liquid character of the product is retained up to the point where the polyoxyethylene content is about 50% of the total weight of the condensation product, which corresponds to condensation with up to about 40 moles of ethylene oxide. Examples of compounds of this type include certain of the commercially available Pluronic™ surfactants, marketed by BASF.

Also not preferred, although suitable for use as nonionic surfactants herein are the condensation products of ethylene oxide with the product resulting from the reaction of propylene oxide and ethylenediamine. The hydrophobic moiety of these products consists of the reaction product of ethylenediamine and excess propylene oxide, and generally has a molecular weight of from about 2,500 to about 3,000. This hydrophobic moiety is condensed with ethylene oxide to the extent that the condensation product contains from about 40% to about 80% by weight of polyoxyethylene and has a molecular weight of from about 5,000 to about 11,000. Examples of this type of nonionic surfactant include certain of the commercially available Tetronic™ compounds, marketed by BASF.

### Anionic surfactants:

Suitable anionic surfactants for use herein are those having an HLB of less than 12 and according to the following description:

Suitable alkyl sulphonates for use herein include water-insoluble salts or acids of the formula RSO₃M wherein R is a C₆-C₂₀ linear or branched, saturated or unsaturated alkyl group, preferably a C₁₂-C₁₈ alkyl group and more preferably a C₁₄-C₁₆ alkyl group, and M is H or a cation, e.g., an alkali metal cation (e.g., sodium, potassium, lithium), or ammonium or substituted ammonium (e.g., methyl-, dimethyl-, and trimethyl ammonium cations and quaternary ammonium cations, such as tetramethyl-ammonium and dimethyl piperidinium cations and quaternary ammonium cations derived from alkylamines such as ethylamine, diethylamine, triethylamine, and mixtures thereof, and the like).

Suitable alkyl aryl sulphonates for use herein include water-insoluble salts or acids of the formula RSO₃M wherein R is an aryl, preferably a benzyl, substituted by a C₆-C₂₀ linear or branched saturated or unsaturated alkyl group, preferably a C₁₂-C₁₈ alkyl group and more preferably a C₁₄-C₁₆ alkyl group, and M is H or a cation, e.g., an alkali metal cation (e.g., sodium, potassium, lithium, calcium, magnesium and the like) or ammonium or substituted ammonium (e.g., methyl-, dimethyl-, and trimethyl ammonium cations and quaternary ammonium cations, such as tetramethyl-ammonium and dimethyl piperidinium cations and quaternary ammonium cations derived from alkylamines such as ethylamine, diethylamine, triethylamine, and mixtures thereof, and the like).

An example of a C14-C16 alkyl sulphonate is Hostapur ® SAS available from Hoechst. An example of commercially available alkyl aryl sulphonate is Lauryl aryl sulphonate from Su.Ma. Particularly preferred alkyl aryl sulphonates are alkyl benzene sulphonates commercially available under trade name Nansa® available from Albright&Wilson.

Suitable alkyl sulphate surfactants for use herein are according to the formula R₁SO₄M wherein R₁ represents a hydrocarbon group selected from the group consisting of straight or branched alkyl radicals containing from 6 to 20 carbon atoms and alkyl phenyl radicals containing from 6 to 15 carbon atoms in the alkyl group. M is H or a cation, e.g., an alkali metal cation (e.g., sodium, potassium, lithium, calcium, magnesium and the like) or ammonium or substituted ammonium (e.g., methyl-, dimethyl-, and trimethyl ammonium cations and quaternary ammonium cations, such as tetramethyl-ammonium and dimethyl piperidinium cations and quaternary ammonium cations derived from alkylamines such as ethylamine, diethylamine, triethylamine, and mixtures thereof, and the like).

Suitable alkyl alkoxylated sulphate surfactants for use herein are according to the formula RO(A)ₘSO₃M wherein R is an unsubstituted C₅-C₂₀ alkyl or hydroxyalkyl group having a C₅-C₂₀ alkyl component, preferably a C₁₂-C₂₀ alkyl or hydroxyalkyl, more preferably C₅-C₁₅ alkyl or hydroxyalkyl, A is an ethoxy or propoxy unit, m is greater than zero, typically between about 0.5 and about 10, more preferably between about 3 and about 7 and M is H or a cation which can be, for example, a metal cation (e.g., sodium, potassium, lithium, calcium, magnesium, etc.), ammonium or substituted-ammonium cation. Alkyl ethoxylated sulfates as well as alkyl propoxylated sulfates are contemplated herein. Specific examples of substituted ammonium cations include trimethyl-ammonium and quaternary ammonium cations, such as tetramethyl-ammonium, dimethyl piperidinium and cations derived from alkanolamines such as ethylamine, diethylamine, triethylamine, mixtures thereof, and the like. Exemplary surfactants are C₁₂-C₁₈E (3.0), and C₁₂-C₁₈ alkyl polyethoxylate (4.0) sulfate C₁₂-C₁₈E(12.0)M, wherein M is conveniently selected from sodium and potassium.

Suitable C₆-C₂₀ alkyl alkoxylated linear or branched diphenyl oxide disulphonate surfactants for use herein are according to the following formula: 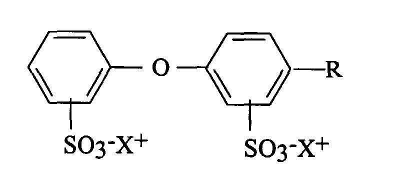 wherein R is a C₆-C₂₀ linear or branched, saturated or unsaturated alkyl group, preferably a C₆-C₁₈ alkyl group and more preferably a C₆-C₁₀ alkyl group, and X+ is H or a cation, e.g., an alkali metal cation (e.g., sodium, potassium, lithium, calcium, magnesium and the like). Particularly suitable C₆-C₂₀ alkyl alkoxylated linear or branched diphenyl oxide disulphonate surfactants to be used herein are the C12 branched di phenyl oxide disulphonic acid and C16 linear di phenyl oxide disulphonate sodium salt respectively commercially available by DOW under the trade name Dowfax 2A1® and Dowfax 8390®.

Other anionic surfactants useful herein include salts (including, for example, sodium, potassium, ammonium, and substituted ammonium salts such as mono-, di- and triethanolamine salts) of soap, C₈-C₂₄ olefinsulfonates, sulfonated polycarboxylic acids prepared by sulfonation of the pyrolyzed product of alkaline earth metal citrates, e.g., as described in British patent specification No. 1,082,179, C₈-C₂₄ alkylpolyglycolethersulfates (containing up to 10 moles of ethylene oxide); alkyl ester sulfonates such as C₁₄₋₁₆ methyl ester sulfonates; acyl glycerol sulfonates, fatty oleyl glycerol sulfates, alkyl phenol ethylene oxide ether sulfates, alkyl phosphates, isethionates such as the acyl isethionates, N-acyl taurates, alkyl succinamates and sulfosuccinates, monoesters of sulfosuccinate (especially saturated and unsaturated C₁₂-C₁₈ monoesters) diesters of sulfosuccinate (especially saturated and unsaturated C₆-C₁₄ diesters), acyl sarcosinates, sulfates of alkylpolysaccharides such as the sulfates of alkylpolyglucoside (the nonionic nonsulfated compounds being described below), alkyl polyethoxy carboxylates such as those of the formula RO(CH₂CH₂O)ₖCH₂COO-M⁺ wherein R is a C₈-C₂₂ alkyl, k is an integer from 0 to 10, and M is a soluble salt-forming cation. Resin acids and hydrogenated resin acids are also suitable, such as rosin, hydrogenated rosin, and resin acids and hydrogenated resin acids present in or derived from tall oil. Further examples are given in "Surface Active Agents and Detergents" (Vol. I and II by Schwartz, Perry and Berch). A variety of such surfactants are also generally disclosed in U.S. Patent 3,929,678, issued December 30, 1975 to Laughlin, et al. at Column 23, line 58 through Column 29, line 23.

Preferred anionic surfactants for use herein are the alkyl sulphate surfactants as described herein before. Suitable alkyl sulphate is for instance Lauryl sodium sulphate commercially available from Henkel corp. under the name Avirol SL11®

### Zwitterionic surfactants

Suitable zwitterionic surfactants to be used herein contain both cationic and anionic hydrophilic groups on the same molecule at a relatively wide range of pH's. The typical cationic group is a quaternary ammonium group, although other positively charged groups like phosphonium, imidazolium and sulfonium groups can be used. The typical anionic hydrophilic groups are carboxylates and sulfonates, although other groups like sulfates, phosphonates, and the like can be used.

Preferred zwitterionic surfactants for use herein are quaternary ammonium surfactants according to the formula R₁ R₂R₃R₄N⁺ X⁻, wherein X is a counteranion such as halogen, methyl sulphate, methyl sulphonate, or hydroxide, R₁ is a saturated or unsaturated, substituted or unsubstituted, linear or branched alkyl group containing from 1 to 30 carbon atoms, preferably from 8 to 20, more preferably from 12 to 20 and R₂, R₃ and R₄ are independently hydrogen, or saturated or unsaturated, substituted or unsubstituted, linear or branched alkyl groups containing from 1 to 4 carbon atoms, preferably from 1 to 3 and more preferably methyl. In highly preferred quaternary ammonium surfactants herein R₁ is a C₁₀-C₁₈ hydrocarbon chain, most preferably C₁₂, _{C14,}, or C₁₆, and R₂, R₃ and R₄ are all three methyl, and X is halogen, preferably bromide or chloride, most preferably bromide.

Examples of quaternary ammonium surfactants are myristyl trimethylammonium methyl sulphate, cetyl trimethylammonium methyl sulphate, lauryl trimethyl ammonium bromide, stearyl trimethyl ammonium bromide (STAB), cetyl trimethyl ammonium bromide (CTAB) and myristyl trimethyl ammonium bromide (MTAB). Highly preferred herein are lauryl and cetyl trimethyl ammonium salts. Such trimethyl quaternary ammonium surfactants may be commercially available from Hoechst, or from Albright & Wilson under the trade name Empigen CM®.

### Optional agents

The articles according to the present invention preferably further comprise other conventional agents or mixtures thereof.

For instance additional odour control agents or combinations thereof, known in the art for this purpose may be used herein. These agents can typically be classified according to the type of odour the agent is intended to combat. Odors may be chemically classified as being acidic, basic or neutral.

Alternatively, the odor control agents may be categorised with respect to the mechanism by which the malodor detection is reduced or prevented. For example, odor control agents which chemically react with malodorous compounds or with compounds which produce malodorous degradation products thereby generating compounds lacking odor or having an odor acceptable to consumers may also be utilized herein.

Suitable odor control agents for use herein typically include carbonates (e.g., sodium carbonate), bicarbonates (e.g., sodium bicarbonate), phosphates (e.g., sodium phosphate), sulphates (e.g., zinc and copper sulphates), carboxylic acids such as citric acid, lauric acid, boric acid, adipic acid and maleic acid, activated carbons, clays, zeolites, silicas, absorbent gelling materials (AGM) and starches. Such odor control agents and systems are disclosed in more details hereinafter and for example in EP-A- 348 978, EP-A- 510 619, WO 91/12029, WO 91/11977, WO 91/12030, WO 81/01643 and WO 96/06589.

Suitable odour control agents also include chelating agents and may be selected from amino carboxylates such as for example ethylenediamine-tetracetate, as described for example in US 4356190, amino phosphonates such as ethylenediaminetetrakis (methylene- phosphonates), polyfunctionally-substituted aromatic chelating agents as described in US 3 812 044 and mixtures thereof. Without intending to be bound by theory it is believed that the benefit of these materials is in part due to their exceptional ability to remove iron, copper, calcium, magnesium and manganese ions present in the absorbed fluids and their degradation products by the formation of chelates.

Another suitable odor control agent for use herein is a buffer system, such as citric acid and sodium bicarbonate, sodium phosphate and sorbic acid buffer systems. Also, buffer systems having a pH of from 7 to 10 as described for example in WO94/25077 may be useful herein.

Alternative odor control agents are ion exchange resins such as those described in US 4 289 513 and US 3340875.

Masking agents such as perfumes may also be used as odor control agents herein.

Typically, the articles according to the present invention, like the disposable absorbent articles, may comprise the additional odour control agent or a mixture thereof at a level of from 0.5 gm⁻² to 600 gm⁻², preferably from 5 to 500 gm⁻², more preferably from 10 gm⁻² to 350 gm⁻² and most preferably from 20 gm⁻² to 200 gm⁻²

### Absorbent gelling odor control materials

As is well-known from recent commercial practice, absorbent gelling materials (sometimes referred to as "super-sorbers") are becoming broadly used in absorbent articles. AGM's are materials which have fluid-absorbing properties.

Such materials are highly preferred herein as the optional odor control agent due to their dual function of absorbing fluids and odors.

Such materials form hydrogels on contact with water (e.g., with urine, blood, and the like). One highly preferred type of hydrogel-forming, absorbent gelling material is based on polyacids, especially polyacrylic acid. Hydrogel- forming polymeric materials of this type are those which, upon contact with fluids (i.e., liquids) such as water or body fluids, imbibe such fluids and thereby form hydrogels. These preferred absorbent gelling materials will generally comprise substantially water-insoluble, slightly cross-linked, partially neutralized, hydrogel-forming polymer materials prepared from polymerizable, unsaturated, acid-containing monomers. In such materials, the polymeric component formed from unsaturated, acid-containing monomers may comprise the entire gelling agent or may be grafted onto other types of polymer moieties such as starch or cellulose. Acrylic acid grafted starch materials are of this latter type. Thus, the preferred absorbent gelling materials include hydrolyzed acrylonitrile grafted starch, acrylic acid grafted starch, polyacrylates, maleic anhydride-based copolymers and combinations thereof. Especially preferred absorbent gelling materials are the polyacrylates and acrylic acid grafted starch.

Whatever the nature of the polymer components of the preferred absorbent gelling materials, such materials will in general be slightly cross-linked. Crosslinking serves to render these preferred hydrogel-forming absorbent materials substantially water-insoluble, and cross-linking also in part determines the gel volume and extractable polymer characteristics of the hydrogels formed therefrom. Suitable cross-linking agents are well known in the art and include, for example, (1) compounds having at least two polymerizable double bonds; (2) compounds having at least one polymerizable double bond and at least one functional group reactive with the acid-containing monomer material; (3) compounds having at least two functional groups reactive with the acid-containing monomer materials; and (4) polyvalent metal compounds which can from ionic cross-linkages. Cross-linking agents of the foregoing types are described in greater detail in Masuda et al; U.S. Patent 4,076,663; Issued February 28, 1978. Preferred cross-linking agents are the di- or polyesters of unsaturated mono-or polycarboxylic acids with polyols, the bisacrylamides and the di-or triallyl amines. Especially preferred cross-linking agents are N,N'-methylenebisacrylamide, trimethylol propane triacrylate and triallyl amine. The cross-linking agent will generally comprise from about 0.001 mole percent to 5 mole percent of the preferred materials. More preferably, the cross-linking agent will comprise from about 0.01 mole percent to 3 mole percent of the gelling materials used herein.

The preferred, slightly cross-linked, hydrogel-forming absorbent gelling materials will generally be employed in their partially neutralized form. For purposes described herein, such materials are considered partially neutralized when at least 25 mole percent, and preferably at least 50 mole percent of monomers used to form the polymer are acid group-containing monomers which have been neutralized with a salt-forming cation. Suitable salt-forming cations include alkali metal, ammonium, substituted ammonium and amines. This percentage of the total monomers utilized which are neutralized acid group-containing monomers is referred to as the "degree of neutralization". Typically, commercial absorbent gelling materials have a degree of neutralization somewhat less than 90%.

The preferred absorbent gelling materials used herein are those which have a relatively high capacity for imbibing fluids encountered in the absorbent articles; this capacity can be quantified by referencing the "gel volume" of said absorbent gelling materials. Gel volume can be defined in terms of the amount of synthetic urine absorbed by any given absorbent gelling agent buffer and is specified as grams of synthetic urine per gram of gelling agent.

Gel volume in synthetic urine (see Brandt, et al, below) can be determined by forming a suspension of about 0.1-0.2 parts of dried absorbent gelling material to be tested with about 20 parts of synthetic urine. This suspension is maintained at ambient temperature under gentle stirring for about 1 hour so that swelling equilibrium is attained. The gel volume (grams of synthetic urine per gram of absorbent gelling material) is then calculated from the weight fraction of the gelling agent in the suspension and the ratio of the liquid volume excluded from the formed hydrogel to the total volume of the suspension. The preferred absorbent gelling materials useful in this invention will have a gel volume of from about 20 to 70 grams, more preferably from about 30 to 60 grams, of synthetic urine per gram of absorbent gelling material.

Another feature of the most highly preferred absorbent gelling materials relates to the level of extractable polymer material present in said materials. Extractable polymer levels can be determined by contacting a sample of preferred absorbent gelling material with a synthetic urine solution for the substantial period of time (e.g., at least 16 hours) which is needed to reach extraction equilibrium, by then filtering the formed hydrogel from the supernatant liquid, and finally by then determining the polymer content of the filtrate. The particular procedure used to determine extractable polymer content of the preferred absorbent gelling agent buffers herein is set forth in Brandt, Goldman and Inglin; U.S. Patent 4,654,039; Issues March 31,1987, Reissue 32,649, The absorbent gelling materials which are especially useful in the absorbent articles herein are those which have an equilibrium extractables content in synthetic urine of no more than about 17%, preferably no more than about 10% by weight of the absorbent gelling material.

The absorbent gelling materials herein before described are typically used in the form of discrete particles. Such absorbent gelling materials can be of any desired shape, e.g., spherical or semi-spherical, cubic, rod-like polyhedral, etc. Shapes having a large greatest dimension/smallest dimension ratio, like needles and flakes, are also contemplated for use herein. Agglomerates of absorbent gelling material particles may also be used.

The size of the absorbent gelling material particles may vary over a wide range. For reason of industrial hygiene, average particle sizes smaller than about 30 microns are less desirable. Particles having a smallest dimension larger than about 2mm may also cause a feeling of grittyness in the absorbent article, which is undesirable from a consumer aesthetics standpoint. Furthermore, rate of fluid absorption can be affected by particle size. Larger particles have very much reduced rates of absorption. Preferred for use herein are absorbent gelling material s particles substantially all of which have a particle size of from about 30 microns to about 2mm. "Particle Size" as used herein means the weighted average of the smallest dimension of the individual particles.

The amount of absorbent gelling material particles typically used in absorbent article will typically range from 0.5 gm⁻² to 250 gm⁻², preferably from 1 to 150 gm⁻², more preferably from 5 gm⁻² to 100 gm⁻² and most preferably from 20 gm⁻² to 70 gm⁻²

### Silica odor control agent

Particularly suitable herein as an additional odor control agent is silica. Silica, i.e. silicon dioxide SiO₂ exists in a variety of crystalline forms and amorphous modifications, any of which are suitable for use herein. In particular, silicas having a high surface area or in agglomerated form are preferred. Silica molecular sieves are not considered to be within the definition of silica as used herein. Preferably the silica is in a highly purified form such that is contains at least 90%, preferably 95%, more preferably 99% silicon dioxide. Most preferably the silica is silica gel having a 100% silica content. Alternatively, the silica may be provided from other sources such as metal silicates including sodium silicate.

In the embodiments of the present invention wherein an hypochlorite bleach or an oxygen bleach releasing chlorine is used like for instance persulphate, it is highly preferred to use silicate salts like sodium silicate as the additional odour control agent. Indeed it has been found that silicate salts are particularly suitable to absorb chlorine odours.

### Zeolite odor control agent

The use and manufacture of zeolite material is well know in the literature and is described in the following reference texts: ZEOLITE SYNTHESIS, ACS Symposium Series 398, Eds. M. L. Occelli and H. E Robson (1989) pages 2-7; ZEOLITE MOLECULAR SIEVES, Structure, Chemistry and Use, by D. W. Breck, John Wiley and Sons (1974) pages 245-250, 313-314 and 348-352; MODERN APPLICATIONS OF MOLECULAR SIEVE ZEOLITES, Ph.D. Dissertation of S. M. Kuznicki, U. of Utah (1980), available from University of Microfilms International, Ann Arbor, Michigan, pages 2-8.

Zeolites are crystalline aluminosilicates of group IA and group IIA elements such as Na, K, Mn, Ca and are chemically represented by the empirical formula :

M_{2/n}O . Al₂O₃. _{y}SiO₂ . wH₂O

where y is 2 or greater, n is the cation valence, and w is the water content in the voids of the zeolite.

Structurally, zeolites are complex, crystalline inorganic polymers based on an infinitely extending framework of AlO₄ and SiO₄ tetrahedra linked to each other by sharing of oxygen ions. This framework structure contains channels or interconnected voids that are occupied by the cations and water molecules.

The structural formula of a zeolite is based on the crystal unit cell, the smallest unit of structure, represented by

M_{x/n} [(AlO₂)x (SiO₂)_{y}] . wH₂O

where n is the valence of cation M, w is the number of water molecules per unit cell, x and y are the total number of tedrahedra per unit cell, y/x usually having values of 1-5.

Zeolites may be naturally derived or synthetically manufactured. The synthetic zeolites being preferred for use herein. Suitable zeolites for use herein include zeolite A, zeolite P, zeolite Y, zeolite X, zeolite DAY, zeolite ZSM-5, or mixtures thereof. Most preferred is zeolite A.

According to the present invention the zeolite is preferably hydrophobic. This is typically achieved by increasing the molar ratio of the SiO₂ to AlO₂ content such that the ratio of x to y is at least 1, preferably from 1 to 500, most preferably from 1 to 6.

### The disposable absorbent article

The odor control system (i.e., the water soluble oxidizing agent, the emulsifier and optional additional odour control agent) may be incorporated into the absorbent article by any of the methods disclosed in the art, for example layered on the core of the absorbent article or mixed within the fibres of the absorbent core.

The odour control system is preferably incorporated between two layers of cellulose tissue. Optionally the system may be bonded between two cellulose tissue layers with, for example, a hot melt adhesive or any suitable bonding system, as described in WO 94/01069.

In one embodiment of the present invention the odour control system is incorporated in a layered structure in accordance with the disclosure of WO 94/01069 or Italian patent application number TO 93A 001028. TO 93A 001028 describes a layered structure substantially as described in WO 94/01069 with the exception that TO 93A 001028 comprises a much higher quantity of absorbent gelling material in the intermediate layer which is between the fibrous layers (120gm⁻²) that would be incorporated as an optional component in the present invention. The intermediate layer comprises in particular a polyethylene powder as thermoplastic material which is mixed with the water soluble oxidizing agent, the emulsifier and optional additional odour control agent. The mixture is then heated such that the polyethylene melts and glues the laminate layers together. Adhesive lines are preferably also placed on the edges of the laminate to ensure that the edges of the laminate stick and any loose oxidising agent as described herein, emulsifier and optional odour control agent present do not fall out of the laminate.

Alternatively, the polyethylene powder may be replaced by a conventional glue for instance those commercially available from ATO Findley under the name H20-31® to glue the laminate layers and/or components together. Advantageously this method step allows to avoid the heating step necessary when using polyethylene powder.

In a preferred embodiement the water soluble oxidizing agent and emulsifier on one side and the optional additional odour control agent if present on the other side are incorporated between two layers of cellulose tissues separated by another layer of cellulose in order to avoid possible reaction between the water soluble oxidizing agent and the additional odour control agent.

The water soluble oxidizing agent, the emulsifier and the optional additional odour control agent may independently be distributed homogeneously or non homogeneously over the entire absorbent article or in at least one layer of the topsheet or in at least one layer of the core or any mixture thereof. The water soluble oxidizing agent, the emulsifier and the optional additional odour control agent may be distributed independently homogeneously or non homogeneously on the whole surface of the desired layer or layers, or on one or several area of the surface layer/layers to which it is positioned (e.g. central area and/or surrounding area like the edges of a layer of the absorbent article) or mixtures thereof. Preferably the water soluble oxidizing agent and the emulsifier are located towards the topsheet or is located in the topsheet itself (preferably the secondary topsheet).

In a preferred embodiement the water soluble oxidizing agent and emulsifier are positioned such that at least a portion of the fluid discharge comes into contact with said water soluble oxidizing agent before the additional odour control agent (e.g., AGM/silica/zeolite) if present. In particular, the water soluble oxidizing agent is located in a separate layer from the additional odour control agent. Preferably the oxidizing agent is located towards the topsheet or is located in the topsheet itself (preferably the secondary topsheet) and the additional odour control agent if present, is located further away from the topsheet than the oxidizing agent. In one embodiment of the present invention, the oxidizing agent is positioned in at least one of the topsheet layers and the additional odour control agent if present is positioned in the core. More preferably, the oxidizing agent is located at the fluid discharge entry point of the absorbent article.

The water soluble oxidizing agent, the emulsifier and the optional odour control agent may be incorporated independently as a powder, a granulate or can be sprayed in the form of for instance an oxidising agent-containing solution within the absorbent article. When used in a granulate or particulate form the oxidizing agent and the emulsifier may be granulated separately and then mixed together or granulated together.

Typical disposable absorbent articles according to the preferred embodiments of the present invention are those as described herein after:

### Absorbent core

According to the present invention, the absorbent can include the following components: (a) an optional primary fluid distribution layer preferably together with a secondary optional fluid distribution layer; (b) a fluid storage layer; (c) an optional fibrous ("dusting") layer underlying the storage layer; and (d) other optional components. According to the present invention the absorbent may have any thickness depending on the end use envisioned.

### a Primary/Secondary Fluid Distribution Layer

One optional component of the absorbent according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilized. The fluid distribution layers can be comprised of any material typical for such distribution layers. In particular fibrous layers maintain the capillaries between fibers even when wet are useful as distribution layers.

### b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials in combination with suitable carriers.

Suitable carriers include materials which are conventionally utilized in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention may comprise multiple layers comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### c Optional Fibrous ("Dusting") Layer

An optional component for inclusion in the absorbent core according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

### d Other Optional Components of the absorbent structure

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

### The topsheet

According to the present invention the absorbent article comprises as an essential component a topsheet. The topsheet may comprise a single layer or a multiplicity of layers. In a preferred embodiment the topsheet comprises a first layer which provides the user facing surface of the topsheet and a second layer between the first layer and the absorbent structure/core.

The topsheet as a whole and hence each layer individually needs to be compliant, soft feeling, and non-irritating to the wearer's skin. It also can have elastic characteristics allowing it to be stretched in one or two directions. According to the present invention the topsheet may be formed from any of the materials available for this purpose and known in the art, such as woven and non woven fabrics and films.

In a preferred embodiment of the present invention at least one of the layers, preferably the upper layer, of the topsheet comprises a hydrophobic, liquid permeable apertured polymeric film. Preferably, the upper layer is provided by a film material having apertures which are provided to facilitate liquid transport from the wearer facing surface towards the absorbent structure. If present the lower layer preferably comprises a non woven layer, an apertured formed film or an airlaid tissue.

The term apertured polymeric topsheet as used herein refers to topsheets comprising at least one layer or a multiplicity of layers wherein at least one layer is formed from a continuous or uninterrupted film material wherein apertures are created. It has been surprisingly discovered that apertured polymeric film topsheets yield significantly better odour control than other types of topsheets such as for example thermal bonded nonwoven materials.

In general the apertured polymeric topsheet of the present invention is compliant, soft feeling, and non-irritating to the wearer's skin. Further, the topsheet is fluid pervious permitting fluids (e.g., menses and/or urine) to readily penetrate through its thickness. Suitable apertured polymeric film topsheets for use herein include polymeric apertured formed films, thermoplastic films, apertured plastic films, and hydroformed thermoplastic films; porous foams; net-like reticulated foams and thermoplastic films; and thermoplastic scrims.

Preferred topsheets for use in the present invention are selected from apertured formed film topsheets. Apertured formed films are especially preferred for the topsheet because they are pervious to body exudates and yet non-absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in U.S. Patent 3,929,135 (Thompson), issued December 30, 1975; U.S. Patent 4,324,246 (Mullane, et al.), issued April 13, 1982; U.S. Patent 4,342,314 (Radel. et al.), issued August 3, 1982; U.S. Patent 4,463,045 (Ahr et al.), issued July 31, 1984; and U.S. 5,006,394 (Baird), issued April 9, 1991. Each of these patents are incorporated herein by reference. Particularly preferred microapertured formed film topsheets are disclosed in U.S. patent 4,609,518 (Curro et al), issue September 2, 1986 and U.S. patent 4,629,643 (Curro et al), issued December 16, 1986, which are incorporated by reference. The preferred topsheet for the present invention is the formed film described in one or more of the above patents and marketed on sanitary napkins by The Procter & Gamble Company of Cincinnati, Ohio as "DRI-WEAVE."

Suitable topsheets in the field of three dimensional formed film are described in EP 0 018 020 and EP 0 059 506. Especially preferred in a three dimensional formed polymeric topsheet having openings in the shape of regular pentagons which are regularly spaced and have an opening of 0.41 square millimeter. The openings are spaced 0.37 square millimeters apart transversely and 0.25 millimeters longitudinally. This topsheet has an initial opening (preforming ) thickness of about 25m a final (post forming) thickness of about 0.53mm and an open area of from 25% to about 40%.

Another formed film topsheet which is especially preferred is one having openings of two shapes; regular pentagons having an area of about 0.21 square millimeters and an irregular hexagon having an area of 1.78 square millimeters. The openings are distributed so that the distance between the sides of the figures is about 0.37 mm to about 0.42mm. The preforming and post forming film thickness are respectively 0.25 and 0.43 mm. This film has an open area of about 33.7%. Both films are made according to the teachings of the above mentioned patents.

A third form suitable topsheet comprises two separate perforated polymeric films superimposed one on the other.

The body surface of the formed film topsheet of the present invention may also be hydrophilic so as to help liquid to transfer through the topsheet faster than if the body surface was not hydrophilic. In this manner the likelihood that menstrual fluid will flow off the topsheet rather than flowing into and being absorbed by the absorbent structure is diminished. In a preferred embodiment, surfactant is incorporated into the polymeric materials of the formed film topsheet such as is described in U.S. Patent Application Serial No. 07/794,745, "Absorbent Article Having A Nonwoven and Apertured Film Coversheet" filed on November 19, 1991 by Aziz, et al., which is incorporated by reference. Alternatively, the body surface of the topsheet can be made hydrophilic by treating it with a surfactant such as is described in the above referenced U.S. 4,950,254, incorporated herein by reference.

### The backsheet

The backsheet primarily prevents the extrudes absorbed and contained in the absorbent structure from wetting articles that contact the absorbent product such as underpants, pants, pyjamas and undergarments. The backsheet is preferably impervious to liquids (e.g. menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet typically extends across the whole of the absorbent structure and can extend into and form part of or all of the preferred sideflaps, side wrapping elements or wings.

The backsheet can comprise a woven or nonwoven material, polymeric films such as thermoplastic films of polyethylene or polypropylene, or composite materials such as a film-coated nonwoven material. Preferably, the backsheet is a polyethylene film typically having a thickness of from about 0.012 mm (0.5 mil) to about 0.051 mm (2.0 mil).

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matt finished to provide a more clothlike appearance. Further, the backsheet can permit vapors to escape from the absorbent structure, i.e. be breathable, while still preventing extrudates from passing through the backsheet. Also breathable backsheets comprising several layers, e.g. film plus non-woven structures, can be used.

Suitable breathable backsheets for use herein include all breathable backsheets known in the art. In principle there are two types of breathable backsheets, single layer breathable backsheets which are breathable and impervious to liquids and backsheets having at least two layers, which in combination provide both breathability and liquid imperviousness.

Suitable single layer breathable backsheets for use herein include those described for example in GB A 2184 389, GB A 2184 390, GB A 2184 391, US 4 591 523, US 3 989 867 US 3 156 242 and European Patent Application number 95120653.1.

Suitable dual or multi layer breathable backsheets for use herein include those exemplified in US 3 881 489, US 4 341 216, US 4 713 068, US 4 818 600, EPO 203 821, EPO 710 471, EPO 710 472, European Patent Application numbers 95120647.3, 95120652.3, 95120653.1 and 96830097.0.

Particularly preferred are backsheets meeting the requirements as defined in European Patent Application number 96830343.8 and more preferably wherein the absorbent article also meets the requirements as described therein.

According to the present invention the breathable backsheet comprises at least one, preferably at least two water vapour permeable layers. Suitable water vapour permeable layers include 2 dimensional, planar micro and macro-porous films, monolithic films, macroscopically expanded films and formed apertured films. According to the present invention the apertures in said layer may be of any configuration, but are preferably spherical or oblong. The apertures may also be of varying dimensions. In a preferred embodiment the apertures are preferably evenly distributed across the entire surface of the layer, however layers having only certain regions of the surface having apertures is also envisioned.

2 dimensional planar films as used herein have apertures having an average diameter of from 5 micrometers to 200 micrometers. Typically, 2-dimensional planar micro porous films suitable for use herein have apertures having average diameters of from 150 micrometers to 5 micrometers, preferably from 120 micrometers to 10 micrometers, most preferably from 90 micrometers to 15 micrometers. Typical 2 dimensional planar macroporous films have apertures having average diameters of from 200 micrometers to 90 micrometers. Macroscopically expanded films and formed apertured films suitable for use herein typically have apertures having diameters from 100 micrometers to 500 micrometers. Embodiments according to the present invention wherein the backsheet comprises a macroscopically expanded film or an apertured formed film, the backsheet will typically have an open area of more than 5%, preferably from 10% to 35% of the total backsheet surface area.

Suitable 2 dimensional planar layers of the backsheet may be made of any material known in the art, but are preferably manufactured from commonly available polymeric materials. Suitable materials are for example GORE-TEX (TM) or Sympatex (TM) type materials well known in the art for their application in so-called breathable clothing. Other suitable materials include XMP-1001 of Minnesota Mining and Manufacturing Company, St. Paul, Minnesota, USA. As used herein the term 2 dimensional planar layer refers to layers having a depth of less than 1mm, preferably less than 0.5mm, wherein the apertures have an average uniform diameter along their length and which do not protrude out of the plane of the layer. The apertured materials for use as a backsheet in the present invention may be produced using any of the methods known in the art such as described in EPO 293 482 and the references therein. In addition, the dimensions of the apertures produced by this method may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have an orifice located at their terminating ends. Preferably said protuberances are of a funnel shape, similar to those described in US 3, 929,135. The apertures located within the plane and the orifices located at the terminating end of protuberance themselves maybe circular or non circular, provided the cross sectional dimension or area of the orifice at the termination of the protuberance is smaller than the cross sectional dimension or area of the aperture located within the garment facing surface of the layer. Preferably said apertured preformed films are uni directional such that they have at least substantially, if not complete one directional fluid transport towards the core. Suitable macroscopically expanded films for use herein include films as described in for example in US 637 819 and US 4 591 523.

Suitable macroscopically expanded films for use herein include films as described in for example US 4 637 819 and US 4 591 523.

Suitable monolithic films include Hytrel™, available from DuPont Corporation, USA, and other such materials as described in Index 93 Congress, Session 7A "Adding value to Nonwovens", J-C. Cardinal and Y. Trouilhet, DuPont de Nemours International S.A., Switzerland.

According to the present invention the backsheet may comprise in addition to said water vapour permeable layer additional backsheet layers. Said additional layers may be located on either side of said water vapour permeable layer of the backsheet. The additional layers may be of any material, such as fibrous layers or additional water vapour permeable layers as described herein above.

### Odor control test

The odor reduction is measured by for example an in vitro sniff test. This test consists in analyzing the products (e.g., pads) (including references) by expert graders that express their judgment about (un)pleasantness of the odor of the products.

The present invention is further illustrated by the following examples.

### Examples:

### Example A

The feminine pads used in the following examples were Always (Always is a registered Trade Mark) as sold by the Procter & Gamble Company.

Each feminine pad was opened by culling the wrap around the perforated coverstock at its bottom face approximately along a longitudinal edge of the release paper which covers the external adhesive layer. The side of the absorbent fibrous core was then exposed by slightly shifting the water impermeable plastic bottom layer and subsequently, the fibrous core was split into two halves, each having approximately the same thickness, along a plane which is parallel to the plane of the napkin itself. The oxidising agent and the emulsifier were homogeneously mixed together to obtain a premix (e.g. in the form of a wet cake). This premix was homogeneously distributed between these two fibrous layers which were then joined together to reconstitute the absorbent core.

The water impermeable inner backsheet was then put back into its original position and the wrap around perforated coverstock was sealed along the cut by means of e.g. a double sided adhesive tape.

Samples were produced using the method above, containing the odor control systems as described hereinbelow.

The oxidising agent (1.0g) used was potassium persulphate commercially available from FLUKA or urea peroxide commercially available from Fluka®. The emulsifier was 0.05g of Neodol®23-6,5 commercially available from Shell.

### Example B

In example B samples were produced using the same method as in Example A, except that the emulsifier used was 0.1g of Dobanol® 23-2 commercially available from SHELL instead of 0.05g of Neodol®23-6,5.

### Example C

In example C samples were produced using the same method as in Example A, wherein an additional odour control agent was homogenously mixed together with the oxidising agent and the emulsifier to obtain the premix. The additional odour control agent used was sodium silicate (1.0g) commercially available from ALDRICH

### Example D

Other pads were prepared by following the method in example A except that after having split the fibrous core into two halves, the premix of oxidising agent and emulsifier as described in example A was homogeneously distributed onto the upper halve fibrous layer (i.e. the fibrous layer halve intended to be closer to the topsheet) and that AGM was homogeneously distributed onto the lower halve fibrous layer (i.e., the one intended to be closer to the backsheet of the pad once reconstituted). Then a layer of airlaid tissue (19 mm* 70 mm of low basis weight) available from Fripa under the code/name NCB Tissue HWS was positioned between the two halve fibrous layers which are then joined together to reconstitute the absorbent core. The presence of the airlaid tissue between the two fibrous layer avoids direct contact between the oxidising agent and the AGM (odor control agent).

The oxidising agent (1.0g) used was persulphate or urea peroxide commercially available from FLUKA ®. The AGM (0.8g) used was XZ 9589001, available from Dow Chemicals. The emulsifier used was 0.05g of Neodol®23-6,5 commercially available from Shell .

### Example E

In example E samples were produced using the same method as in Example D, except that the emulsifier used was 0.1g of Dobanol® 23-2 commercially available from SHELL instead of 0.05g of Neodol®23-6,5.

### Example F

In example F samples were produced using the same method as in Example D, except that sodium silicate (1.0g) commercially available from ALDRICH was added on top of AGM.

All the above pads showed outstanding odor control benefit when coming into contact with bodily fluids.

## Claims

1. An article comprising an odour control system for controlling odours, typically those associated with bodily fluids, characterized in that the odour control system comprises at least a water soluble oxidizing agent together with an emulsifier.

2. An article according to claim 1 wherein said oxidizing agent has a water solubility of more than 0.3 g per 100 ml of water at 25°C and is preferably a hypohalite bleach and/or an oxygen bleach.

3. An article according to any of the preceding claims wherein the oxidizing agent is selected from the group of hypochlorites, hypobromites, hypoiodites, dichloroisocyanurates, trichlorocyanurates, N-chloroimides, N-chloroamides, N-chloroamines, chlorohydantoins, chlorinated trisodium phosphate dodecahydrates, chloramine T, chlorines, chlorites, chlorine dioxides, perchlorates, percarbonates, persilicates, persulphates, perphosphates, perborates, alkyl hydroperoxides, peroxides, periodates, permanganates, alkali metal and alkaline earth metal salts thereof, urea peroxides, ruthenium tetra-oxides, osmium tetra-oxides, chromium compounds, lead compounds, nitrate compounds, metallic salts and mixtures thereof and more preferably is urea peroxide and/or persulphate.

4. An article according to any of the preceding claims which comprises from 1 gm⁻² to 500 gm⁻², preferably from 5 to 250 gm⁻², more preferably from 10 gm⁻² to 100 gm⁻² and most preferably from 20 gm⁻² to 70gm⁻² of a water soluble oxidising agent of mixture thereof.

5. An article according to any of the preceding claims, wherein the emulsifier is a surfactant having an HLB of less than 12 or a mixture thereof, preferably a nonionic surfactant, an anionic surfactant, a zwitterionic surfactant, or a mixture thereof.

6. An article according to any of the preceding claims wherein the emulsifier is a nonionic surfactant having an HLB of less than 12 or a mixture thereof, preferably of less than 10 and is typically selected from the group consisting of alkoxylated nonionic surfactant and mixtures thereof.

7. An article according to any of the preceding claims which comprises from 0.5gm⁻² to 200 gm⁻², preferably from 5 gm⁻² to 100 gm⁻² and more preferably from 10 gm⁻² to 60 gm⁻² of the emulsifier or a mixture thereof.

8. An article according to any of the preceding claims, which further comprises an additional odour control agent typically selected from the group consisting of absorbing gelling materials, silicas, zeolites, carbons, starches, chelating agents, pH buffered materials, chitin, kieselguhr, clays, ion exchange resins, carbonates, bicarbonates, phosphates, sulphates, carboxylic acids and combination thereof and preferably is an absorbing gelling material, a silicate, a zeolite or a combination thereof.

9. An article according to claim 8 which comprises from 0.5 gm⁻² to 600 gm⁻², preferably from 5 to 500 gm⁻², more preferably from 10 gm⁻² to 350 gm⁻² and most preferably from 20 gm⁻² to 200 gm^{-²} of said additional odour control agent or a mixture thereof.

10. An article according to any of the preceding claims wherein said article is a disposable absorbent article, preferably a sanitary napkin, pantiliner, tampon, diaper, incontinent pad, breast pad, perspiration pad or interlabial pad.

11. An article according to any of the preceding claims wherein said article is an absorbent disposable article comprising a liquid pervious topsheet, a backsheet and an absorbent core intermediate said backsheet and said topsheet.

12. The use, as an odour control system, of a water soluble oxidising agent, preferably persulphate and/or urea peroxide, together with an emulsifier, preferably a surfactant with an HLB of less than 12 or a mixture thereof.
